# EUROPEAN PATENT APPLICATION

(11) **EP 4 086 334 A1**
(43) Date of publication of application: **09.11.2022**
(21) Application number: 20908615.6
(22) Date of filing: 29.12.2020
(51) Int. Cl.: C12N 1/20, A61K 35/747, A61K 47/10, A61K 47/26, A61P 3/04, A23L 33/135, A23K 10/16, C12R 1/225

(54) **LACTOBACILLUS FERMENTUM STRAIN, AND COMPOSITION FOR PREVENTING OR TREATING METABOLIC DISEASES CONTAINING SAME**

(30) Priority: 31.12.2019 KR 20190178949
(71) Applicant: GI BIOME, Gyeonggi-do 13201 (KR)
(72) Inventor: YANG, Bo Gie, Seoul 05849 (KR); JANG, Myung Ho, Seoul 05849 (KR); KANG, Chang Ho, Chungju-si Chungcheongbuk-do 27373 (KR); PAEK, Nam Soo, Seoul 06608 (KR)
(74) Representative: HGF
(86) International application number: PCT/KR2020/019344
(87) International publication number: WO 2021/137600

(57) **Abstract**

There is provided a novel *Lactobacillus fermentum* strain (Accession No. KCTC 14105BP) and a composition for preventing or treating a metabolic disease including the same. A *Lactobacillus fermentum* according to the present invention may inhibit fat accumulation in white adipose tissue and increase anti-inflammatory cells. The *Lactobacillus fermentum* strain may exhibit excellent blood glucose improvement effect, and in particular, may reduce a fasting blood glucose level. The *Lactobacillus fermentum* strain may effectively ameliorate insulin resistance by improving glucose tolerance and increasing insulin sensitivity. In addition, the *Lactobacillus fermentum* strain may regulate a concentration of metabolic hormones and adipokines in blood. Therefore, the *Lactobacillus fermentum* strain may be usefully used to prevent and treat a metabolic disease such as obesity.

## Description

### Technical Field

The present disclosure relates to a novel *Lactobacillus fermentum* strain and a composition for preventing or treating a metabolic disease including the same.

### Background Art

As society develops, obesity has emerged as one of the serious diseases, and the World Health Organization (WHO) has recognized obesity as a disease to be treated. Recently, the trend toward increasing prevalence of obesity is also observed in Republic of Korea due to westernized diet, and there is a growing interest in treatment and prevention therefor. Obesity refers to a state of an excessive accumulation of body fat resulting from an imbalance between food consumption and energy expenditure. In addition, obesity is closely related to insulin resistance, glucose tolerance, hyperlipidemia, etc., and may be accompanied by metabolic diseases including cardiovascular diseases, fatty liver diseases, cancers, and diabetes as complications.

Recently, as it became known that the inflammatory response in adipose tissue of an obese patient is increased, obesity is sometimes considered as a low-grade systemic inflammation. In particular, inflammatory response is increased by inflammatory macrophages that are increased in proportion to the size of adipose tissue, inflammatory adipokines produced and secreted by adipose tissue in such a state has been reported as a pathogenic factor of metabolic diseases such as cardiovascular diseases and diabetes. Therefore, obesity which increases these molecules can be seen as a cause of almost all adult diseases.

Currently prescribed obesity treatments are Xenical (Roche Pharmaceuticals., Switzerland), Reductil (Abbott Co., US) and Exolise (Arkopharma LLC, France), or the like. The obesity treatments are largely classified as appetite suppressants, energy expenditure promoters, or fat absorption inhibitors, and most of the obesity treatments are appetite suppressants that suppress appetite by controlling neurotransmitters related to hypothalamus. However, conventional obesity treatments have side effects such as heart diseases, respiratory diseases, and nervous system diseases, and also have a problem in that in vivo persistence thereof is low. Accordingly, there is a need to develop safe and effective obesity treatments.

Meanwhile, probiotics to prevent or treat obesity using safe microorganisms (e.g., lactic acid bacteria) has been actively studied. In particular, research has shown that lactic acid bacteria exhibit effects such as maintenance of normal intestinal flora, improvement of intestinal flora, antidiabetic and antilipidemic effects, inhibition of carcinogenesis, inhibition of colon cancer, and nonspecific activity of the host's immune system.

Regarding lactic acid bacteria related to obesity prevention and treatment effect, KR Patent No. 10-1494279 discloses a *Lactobacillus plantarum* KY1032 strain (Accession No. KCCM-10430) having an inhibitory effect on adipocyte differentiation, and KR Patent No. 10-0996577 discloses a *Lactobacillus curvatus* HY7601 (Accession No. KCTC 11456BP) having obesity inhibitory effect, and KR Patent No. 10-1394348 discloses a *Lactobacillus plantarum* DSR920 strain (Accession No. KCCM 11210P) having an inhibitory effect on adipocyte differentiation, but none of them is mature enough to obtain commercial success.

Therefore, there is a need to continue research on a new strain having excellent anti-obesity effect.

### Detailed Description of the Invention

### Technical Problem

Accordingly, the present inventors found out that a *Lactobacillus fermentum* strain (Accession No. KCTC 14105BP) exhibits excellent anti-obesity effect as a result of researching to develop a new strain with excellent anti-obesity effect, and thereby have completed the present invention.

### Solution to Problem

An aspect of the present invention provides a *Lactobacillus fermentum* strain (Accession No. KCTC 14105BP).

Another aspect of the present invention provides a pharmaceutical composition for preventing or treating a metabolic disease including, as an active ingredient, a *Lactobacillus fermentum* strain (Accession No. KCTC 14105BP) or a culture thereof.

Yet another aspect of the present invention provides a food composition for preventing or inhibiting a metabolic disease including a *Lactobacillus fermentum* strain (Accession No. KCTC 14105BP) or a culture thereof.

Still another aspect of the present invention provides a feed composition for preventing or inhibiting a metabolic disease including a *Lactobacillus fermentum* strain (Accession No. KCTC 14105BP) or a culture thereof.

Yet still another aspect of the present invention provides a method of preventing and treating a metabolic disease including administering a *Lactobacillus fermentum* strain (Accession No. KCTC 14105BP) or a culture thereof to an individual.

### Effect of the Invention

The present disclosure relates to a novel *Lactobacillus fermentum* strain (Accession No. KCTC 14105BP) and a composition for preventing or treating a metabolic disease including the same. *Lactobacillus fermentum* according to the present invention may inhibit fat accumulation in white adipose tissue and increase anti-inflammatory cells. *The Lactobacillus fermentum* strain may exhibit excellent blood glucose improvement effect, and in particular, may reduce a fasting blood glucose level. The *Lactobacillus fermentum* strain may effectively ameliorate insulin resistance by improving glucose tolerance and increasing insulin sensitivity. In addition, the *Lactobacillus fermentum* strain may regulate the concentration of metabolic hormones and adipokines in blood. Therefore, the *Lactobacillus fermentum* strain may be usefully used to prevent and treat a metabolic disease such as obesity.

### Brief Description of Drawings

Exemplary embodiments can be understood in more detail from the following description taken in conjunction with the accompanying drawings, in which:

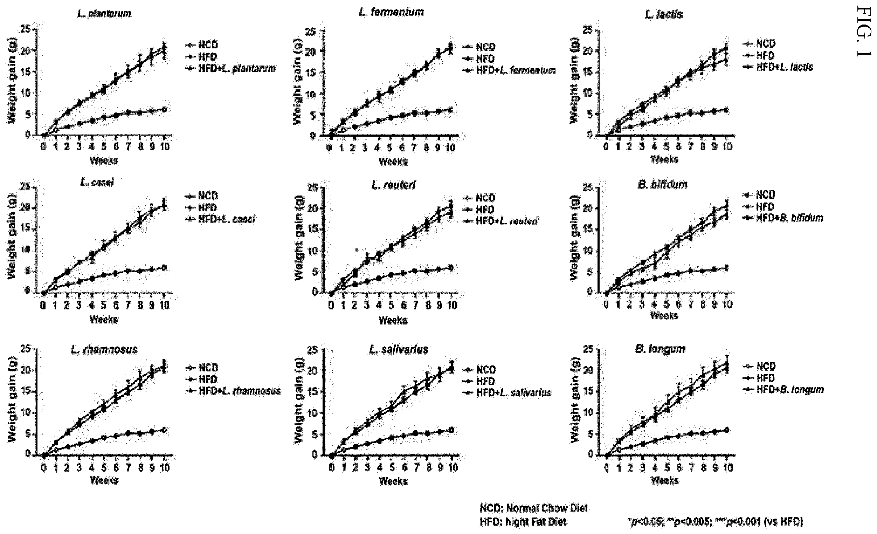

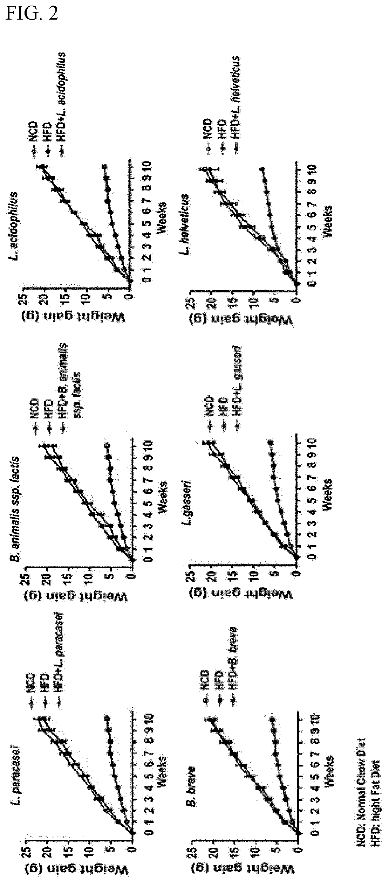

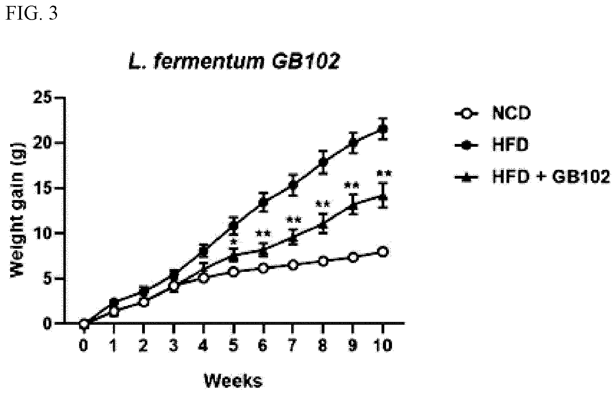

FIGS. 1 to 3 are graphs showing the body weight of the mice fed with high fat diet after oral administration of 16 types of lactic acid bacteria, respectively, compared to the mice only fed with high fat diet;

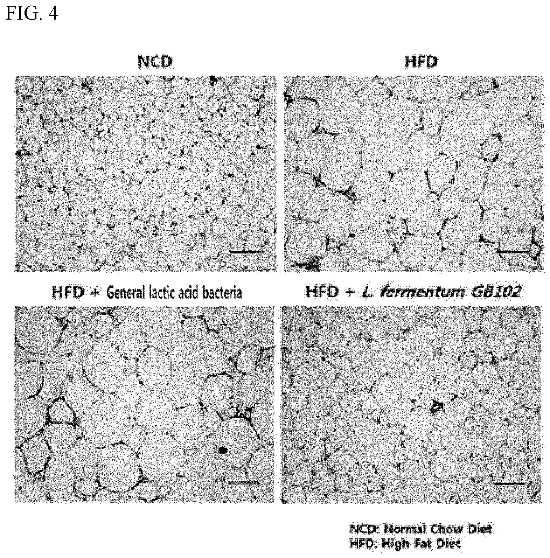

FIG. 4 is a view comparing the size of the adipocytes in white adipose tissue each from the mouse fed with normal chow diet, the mouse fed with high fat diet, the mouse fed with high fat diet and oral administration of general lactic acid bacteria, and the mouse fed with high fat diet and an oral administration of *L. fermentum GB102* strain to compare;

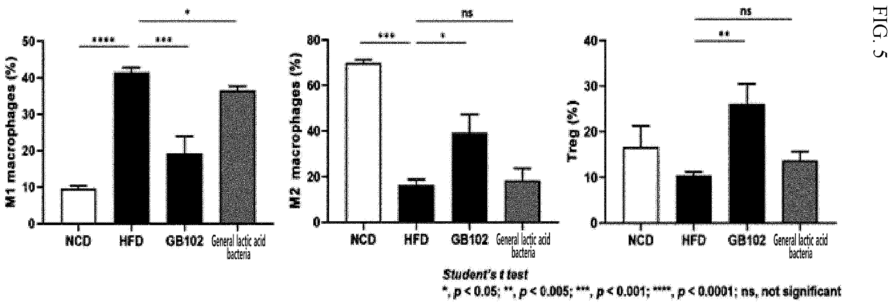

FIG. 5 is graphs comparing the number of M1 macrophages, M2 macrophages and regulatory T (Treg) cells in white adipose tissue of the mouse fed with normal chow diet, the mouse fed with high fat diet, the mouse fed with high fat diet and oral administration of general lactic acid bacteria, and the mouse fed with high fat diet and oral administration of a *L. fermentum GB102* strain;

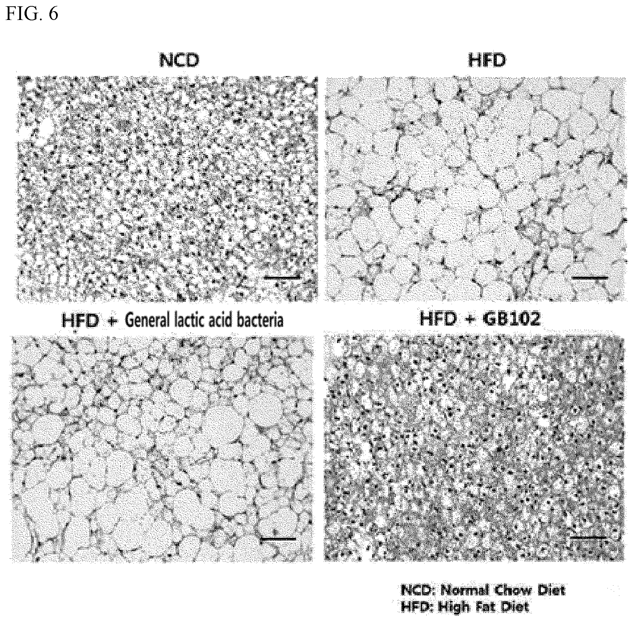

FIG. 6 is a view comparing the size of the adipocytes in brown adipose tissue of the mouse fed with normal chow diet, the mouse fed with high fat diet, the mouse fed with high fat diet and oral administration of general lactic acid bacteria, and the mouse fed with high fat diet and oral administration of a *L. fermentum GB102* strain;

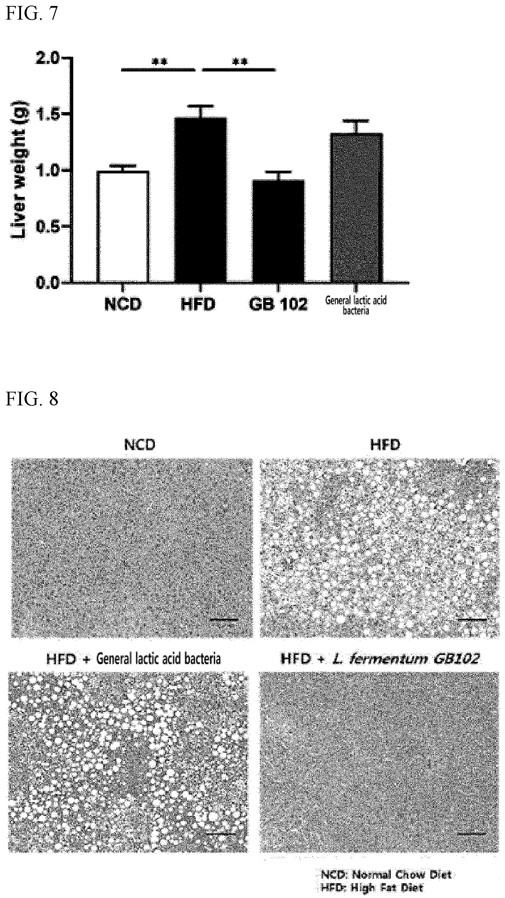

FIG. 7 is a graph comparing the liver weight of the mouse fed with normal chow diet, the mouse fed with high fat diet, the mouse fed with high fat diet and oral administration of general lactic acid bacteria, and the mouse fed with high fat diet and an oral administration of a *L. fermentum GB102* strain;
FIG. 8 is a view comparing the degree of fat accumulation in liver tissue of the mouse fed with normal chow diet, the mouse fed with high fat diet, the mouse fed with high fat diet and oral administration of general lactic acid bacteria, and the mouse fed with high fat diet and an oral administration of a *L. fermentum GB102*;

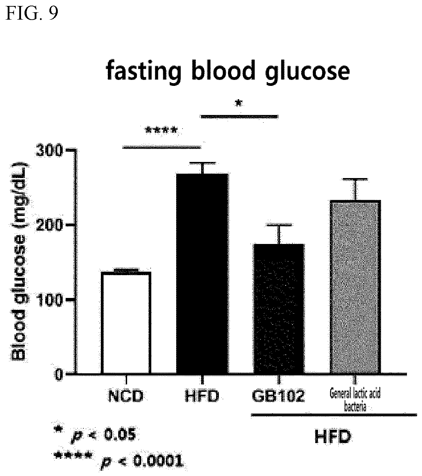

FIG. 9 is a graph comparing the fasting blood glucose level of the mouse fed with normal chow diet, the mouse fed with high fat diet, the mouse fed with high fat diet and oral administration of general lactic acid bacteria, and the mouse fed with high fat diet and an oral administration of a *L. fermentum GB102* strain to confirm blood glucose-lowering effect;

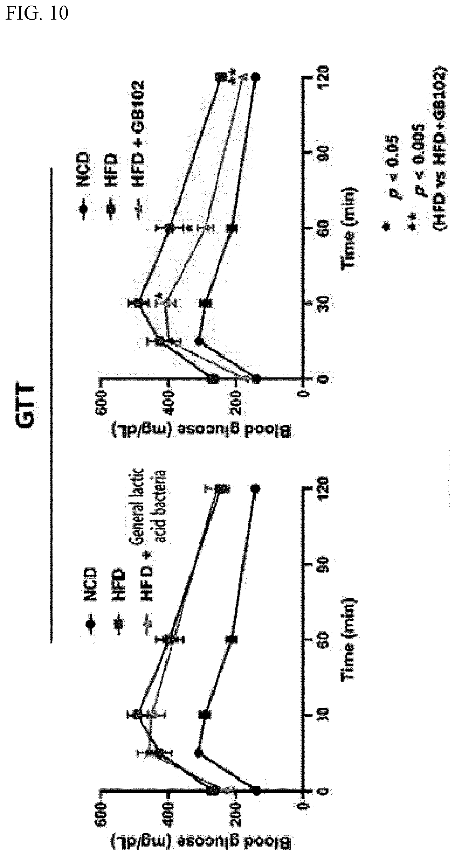

FIG. 10 is graphs comparing the blood glucose level after administration of glucose over time in the mouse fed with normal chow diet, the mouse fed with high fat diet, the mouse fed with high fat diet and oral administration of general lactic acid bacteria, and the mouse fed with high fat diet and an oral administration of a *L. fermentum GB102* strain to confirm glucose tolerance improvement effect;

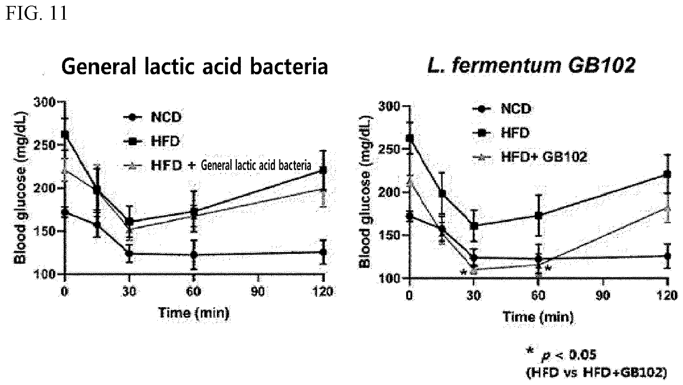

FIG. 11 is graphs comparing the blood glucose level after administration of insulin over time in the mouse fed with normal chow diet, the mouse fed with high fat diet, the mouse fed with high fat diet and oral administration of general lactic acid bacteria, and the mouse fed with high fat diet and an oral administration of a *L. fermentum* GB102 strain to confirm insulin resistance-ameliorating effect; and

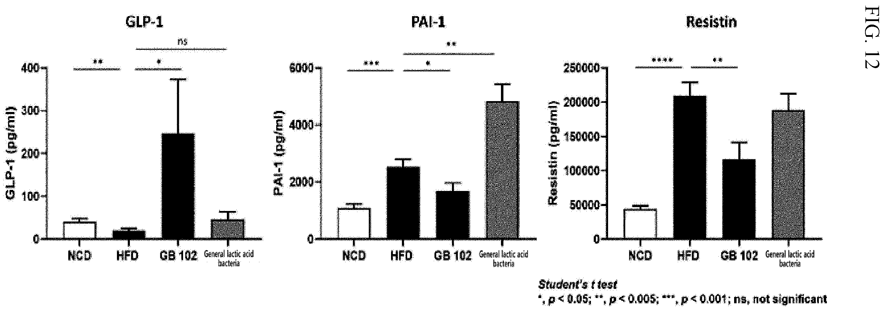

FIG. 12 is graphs comparing the concentrations of glucagone-liked peptide-1 (GLP-1), plasminogen activator inhibitor-1 (PAI-1), and resistin in blood of the mouse fed with normal chow diet, the mouse fed with high fat diet, the mouse fed with high fat diet and oral administration of general lactic acid bacteria, and the mouse fed with high fat diet and an oral administration of a *L. fermentum GB102* strain to confirm the ability to regulate metabolic hormones and adipokines.

### Best Mode for Carrying out the Invention

An aspect of the present invention provides a *Lactobacillus fermentum* strain (Accession No. KCTC 14105BP).

*Lactobacillus* is an aerobic or facultative anaerobic, gram-positive bacillus widely distributed in nature. Genus *Lactobacillus* includes *L. fermentum*, *L. sakei*, etc. The present inventors selected a novel *Lactobacillus fermentum* strain having excellent anti-obesity effect, and named it *"Lactobacillus fermentum GB102.*" The strain was deposited with the Korea Collection for Type Cultures, Korea Research Institute of Bioscience and Biotechnology under the Accession No. SD1335 on September 6, 2019. The same strain was deposited with the Korea Collection for Type Cultures, Korea Research Institute of Bioscience and Biotechnology under the Accession No. KCTC 14105BP on January 14, 2020. In addition, the strain belongs to a probiotic strain, is harmless to the human body, and may be used without side effects.

As used herein, the term "*Lactobacillus fermentum GE102*" is interchangeably described as *L. fermentum GB102* or *Lactobacillus fermentum* strain (Accession No. KCTC 14105BP).

Another aspect of the present invention provides a pharmaceutical composition for preventing or treating a metabolic disease including, as an active ingredient, a *Lactobacillus fermentum* strain (Accession No. KCTC 14105BP) or a culture thereof.

The *Lactobacillus fermentum* strain (Accession No. KCTC 14105BP) is the same as described above. In this case, the strain may be alive or dead, and alive strain is preferred. In addition, a culture of the strain may or may not contain the strain, and it is preferred to contain the strain.

The composition includes, based on the total weight of the composition, a therapeutically effective amount, or a nutritionally effective concentration of the active ingredient *Lactobacillus fermentum* strain (Accession No. KCTC 14105BP) or a culture thereof, wherein a content of 10⁴ to 10¹⁶ CFU/g, preferably 10⁶ to 10¹² CFU/g is included, or a culture having an equivalent number of alive strains is included. In general, for an adult patient, 1×10⁶ CFU/g or more of the alive strain, preferably 1×10⁸ to 1×10¹² CFU/g of the alive strain may be administered once or divided in several times.

As used herein, the term "metabolic disease", also referred to as metabolic syndrome, is a disease assumed to be caused by insulin resistance and is a symptom having abnormality in two or more of cholesterol, blood pressure, and blood glucose levels. Metabolic syndrome is a conceptualization of the phenomenon that the risk factors of various cardiovascular diseases and type 2 diabetes are clustered together as one disease group, and encompasses insulin resistance and all of complex and various metabolic abnormalities and clinical features related to insulin resistance. Specifically, metabolic disease may be any one selected from the group consisting of obesity, hypertension, arteriosclerosis, hyperlipidemia, fatty liver, non-alcoholic fatty liver disease, hyperinsulinemia, diabetes, and insulin resistance syndrome.

As used herein, the term "obesity", also referred to as adipositas, is a disease in which excess body fat has accumulated abnormally. Irregular eating habits, excessive food intake, lack of physical activity, endocrine system diseases, genetic factors, psychological factors, medication, etc. can cause obesity. In addition, obesity increases the incidence of arteriosclerosis, cardiovascular diseases (stroke and ischemic cardiovascular diseases), hypertension, diabetes, hyperlipidemia, fatty liver, cholelithiasis, obstructive sleep apnea, menstrual irregularities, polycystic ovary diseases, infertility, decreased libido, depression, degenerative arthritis, gout, or the like. The obesity may be simple obesity, symptomatic obesity, childhood obesity, adult obesity, cell proliferative obesity, cell hypertrophic obesity, upper body obesity, lower body obesity, visceral fat obesity, or subcutaneous fat obesity.

As used herein, the term "hypertension" refers to a phenomenon in which perfusion blood pressure of blood flow through an artery increases. If the systolic blood pressure reaches 140 mmHg and the diastolic blood pressure reaches 90 mmHg or more, one may generally be diagnosed with hypertension. Hypertension has no noticeable symptoms, and there are primary (or essential) hypertension which does not have a known cause, and secondary hypertension which is caused by renal disease, endocrine disease, pregnancy addiction, or the like. Most cases of hypertension (90 to 95%) are primary hypertension assumed to be caused by genetic causes combined with environmental factors such as obesity, stress, alcohol, and smoking.

As used herein, the term "arteriosclerosis" is defined as a phenomenon of narrowing the width of the arterial wall by loss of the elasticity of the walls of arteries, proliferation of abnormal tissue, and accumulation of fat inside the lining of the artery wall. Arteriosclerosis is a term that refers to a pathological change in an artery, and is named depending on the organs affected by arteriosclerosis. For example, arteriosclerosis includes, but is not limited to, cerebral infarction due to arteriosclerosis, and myocardial infarction due to coronary atherosclerosis.

As used herein, the term "hyperlipidemia" is a disease caused by large amount of lipids such as triglycerides and cholesterol in blood because lipid metabolism is not properly performed. Specifically, hyperlipidemia refers to a state in which lipid components such as triglycerides, LDL cholesterol, and free fatty acids in blood are increased. Hyperlipidemia includes, but is not limited to, hypercholesterolemia or hypertriglyceridemia.

As used herein, the term "fatty liver" is a state of excessive accumulation of fat in hepatocytes due to lipid metabolism disorder, and is defined as a case when fat reaches 5% or more of the liver weight. This causes various diseases such as angina pectoris, myocardial infarction, stroke, arteriosclerosis, fatty liver, pancreatitis, or the like. Fatty liver is divided into alcoholic fatty liver that is caused by alcohol consumption and non-alcoholic fatty liver disease (NAFLD) that is not caused by alcohol.

As used herein, the term "non-alcoholic fatty liver disease" refers to a case when fatty liver is not caused by alcohol, and includes a series of process of liver damage ranging from simple steatosis in liver, non-alcoholic steatohepatitis (NASH) to hepatic cirrhosis. The causes of non-alcoholic fatty liver disease are side effects of drugs such as antiarrhythmic agents, antiviral agents, steroids, cytotoxic drugs; excessive calorie consumption such as carbohydrates; obesity; diabetes; and some genetic causes.

As used herein, the term "hyperinsulinemia" is a disease in which excessive amount of insulin is present in blood. Insulin is a hormone that regulates blood glucose level secreted by the pancreas and promotes the influx of glucose into muscle and other peripheral tissues. Therefore, hyperinsulinemia may occur due to disorder of the pancreas which is an insulin-secreting organ, and is most often caused by insulin resistance.

As used herein, the term "diabetes" is a chronic disease characterized by relative or absolute lack of insulin resulting in glucose-intolerance. The diabetes includes all types of diabetes, and may be, for example, type 1 diabetes, type 2 diabetes, and inherited diabetes, but is not limited thereto. Type 1 diabetes is insulin-dependent diabetes, mainly resulting from destruction of β-cells. As used herein, the term "type 2 diabetes" is insulin-independent diabetes, which is caused by insulin resistance. Type 2 diabetes is caused because increase in insulin is not detected in muscle and adipose tissue, or the action of insulin does not occur effectively even if it is detected.

As used herein, the term "insulin resistance" refers to a state in which cells do not respond to insulin which lower a blood glucose level, and thus cannot effectively burn glucose. When insulin resistance is high, the body thinks more insulin is needed and produces more insulin. This results in hyperinsulinemia, hypertension, or dyslipidemia, as well as heart disease and diabetes.

As used herein, the term "insulin resistance syndrome" is a generic term for the diseases caused by insulin resistance. This is characterized by cell resistance to insulin action, hyperinsulinemia and increased very low-density lipoprotein (VLDL) and triglyceride, and decreased high density lipoprotein (HDL), hypertension, etc., and is recognized as a risk factor for cardiovascular diseases and type 2 diabetes.

According to an embodiment of the present invention, the strain may inhibit fat accumulation in white adipose tissue. Specifically, in an embodiment of the present invention, oral administration of the strain to the mouse model fed with high fat diet resulted in that the size of the adipocytes in white adipose tissue of the mouse model was significantly decreased. Meanwhile, when general lactic acid bacteria were orally administered to the mouse model fed with high fat diet, the size of the adipocytes in white adipose tissue of the mouse model was not decreased. Based on this, it was confirmed that the strain inhibits fat accumulation in white adipose tissue (FIG. 4).

According to an embodiment of the present invention, the strain may reduce meta-inflammation in white adipose tissue. As used herein, the term "meta-inflammation" is chronic and low-grade inflammation, which refers to an inflammatory response that occurs when excessive amount of nutrients or metabolites is provided. In particular, the chronic inflammatory response caused by obesity is known to play an important role in the process of increasing insulin resistance and developing metabolic abnormalities.

In an embodiment of the present invention, oral administration of the strain to the mouse model fed with high fat diet resulted in that M1 macrophages in white adipose tissue of the mouse model decreased, and M2 macrophages and Treg cells were significantly increased. Meanwhile, when general lactic acid bacteria were orally administered to the mouse model fed with high fat diet, amounts of M1 macrophages in white adipose tissue of the mouse model were significantly reduced, but amounts of M2 and Treg cells were not changed. Based on this, it was confirmed that the strain inhibits inflammatory response in white adipose tissue (FIG. 5).

According to an embodiment of the present invention, the strain may inhibit fat accumulation in brown adipose tissue. Specifically, in an embodiment of the present invention, oral administration of the strain to the mouse model fed with high fat diet resulted in that the size of the adipocytes in brown adipose tissue of the mouse model was significantly decreased. Meanwhile, when general lactic acid bacteria were orally administered to the mouse model fed with high fat diet, the size of the adipocytes in brown adipose tissue of the mouse model was not decreased. Based on this, it was confirmed that the strain inhibits fat accumulation in brown adipose tissue (FIG. 6).

According to an embodiment of the present invention, the strain may reduce liver weight and inhibit fat accumulation in liver tissue. Specifically, in an embodiment of the present invention, oral administration of the strain to the mouse model fed with high fat diet resulted in that the liver weight and fat accumulation in tissue of the mouse model were decreased. Meanwhile, when general lactic acid bacteria were orally administered to the mouse model fed with high fat diet, a lot of fat was accumulated in liver tissue of the mouse model and there was no change in weight as well. Based on this, it was confirmed that the strain reduces the liver weight and inhibits fat accumulation in tissue (FIGS. 7 and 8).

According to an embodiment of the present invention, the strain may reduce a blood glucose level. Preferably, the strain may reduce a fasting blood glucose level and improve glucose tolerance. Specifically, in an embodiment of the present invention, oral administration of the strain to the mouse model fed with high fat diet resulted in that a fasting blood glucose level and a blood glucose level after administration of glucose were decreased in the mouse model. Meanwhile, when general lactic acid bacteria were orally administered to the mouse model fed with high fat diet, a fasting blood glucose level and a blood glucose level after administration of glucose were not decreased. Based on this, it was confirmed that the strain reduces a fasting blood glucose level and improves glucose tolerance (FIGS. 9 and 10).

According to an embodiment of the present invention, the strain may ameliorate insulin resistance. Specifically, in an embodiment of the present invention, when the strain was orally administered to the mouse model fed with high fat diet, the blood glucose level was significantly decreased in the mouse model after administration of insulin. Meanwhile, when general lactic acid bacteria were orally administered to the mouse model fed with high fat diet, the fasting blood glucose level and the blood glucose level after administration of insulin were not decreased. Based on this, it was confirmed that the strain ameliorates insulin resistance by increasing insulin sensitivity (FIG. 11).

According to an embodiment of the present invention, the strain may regulate the secretion of metabolic hormones and adipokines. Specifically, the strain may regulate the secretion of metabolic hormones GLP-1, and adipokines PAI-1, and resistins.

As used herein, the term "metabolic hormone" refers to a hormone involved in metabolic regulation, and may preferably be incretin. As used herein, the term "incretin" is a hormone that is secreted in the gastrointestinal tract due to nutrients of food after eating food, and serves to promote the secretion of insulin in a blood glucose-dependent way in the pancreas. Incretins include GLP-1 and glucose-dependent insulinotropic peptide (GIP). The "GLP-1" acts on the pancreas to increase insulin secretion and decrease glucagon secretion, thereby exhibiting blood glucose-lowering effect. In addition, GLP-1 delays gastric emptying, suppresses appetite, and improves the function of β-cells to exhibit anti-diabetic and anti-obesity effects.

As used herein, the term "adipokine" is defined as a soluble mediator that is primarily secreted in adipocytes and exhibits biological action in autocrine, paracrine, or endocrine ways. For example, adipokines includes, but are not limited to, leptin, adiponectin, resistin, tumor necrosis factor-α (TNF-α), interleukin-6, monocyte chemoattractant protein-1 (MCP-1), PAI-1, angiotensinogen, apelin, eotaxin, omentin, virstatin, retinol binding protein 4 (RBP4), adipocyte fatty acid binding protein (A-FABP), lipocalin-2, or the like. Preferably, adipokines may be PAI-1 and resistin.

The PAI-1 is an enzyme that binds to a plasminogen activator to inhibit dissolution of blood, and is known to increase in obesity. In addition, the PAI-1 is known to induce the onset of obesity-related complications, such as atherothrombosis, insulin resistance, and type 2 diabetes. The resistin is known as an adipose tissue-specific secreted factor (ADSF) or a found in inflammatory zone (FIZZ3), and is increased in obesity. The resistin directly induces insulin resistance in muscle and liver, and for example, increases glucose production in the liver. In particular, the resistin contributes to the activation of endothelial cells by promoting expression and secretion of endothelin-1 (ET-1) in inflammation.

In an embodiment of the present invention oral administration of the *GB102* strain to the mouse model fed with high fat diet resulted in an increase in the concentration of GLP-1 in blood which was decreased in the high fat diet mouse model, whereas the concentration of PAI-1 and resistin which was increased by the high fat diet were decreased. However, when general lactic acid bacteria were orally administered to the mouse model fed with high fat diet, changes in the concentration of GLP-1, PAI-1, and resistin in blood were not observed. Based on this, it was confirmed that the strain regulates the concentration of GLP-1, PAI-1 and resistin in blood (FIG. 12).

The composition may further include a cryoprotectant or an excipient. Specifically, the cryoprotectant may be one or more selected from the group consisting of glycerol, trehalose, maltodextrin, skim milk powder, and starch. In addition, the excipient may be one or more selected from the group consisting of glucose, dextrin, and skim milk powder.

The composition may include, based on the total weight of the composition, 0.01 wt% to 20 wt%, for example, 0.01 wt% to 10 wt% of the cryoprotectant, and specifically, the composition may include, 5 wt% to 20 wt% of the glycerol, 2 wt% to 10 wt% of the trehalose, 2 wt% to 10 wt% of the maltodextrin, 0.5 wt% to 2 wt% of the skim milk powder, and 0.1 wt% to 1 wt% of the starch. In addition, the composition may include, based on the total weight of the composition, 75 wt% to 95 wt% or 85 wt% to 95 wt% of the excipient.

Yet another aspect of the present invention provides a food composition for preventing or inhibiting a metabolic disease including a *Lactobacillus fermentum* strain (Accession No. KCTC 14105BP) or a culture thereof.

The *Lactobacillus fermentum* strain (Accession No. KCTC 14105BP) is the same as described above.

In this case, a metabolic disease is as described above, and in particular, the strain may be a food composition for preventing or inhibiting obesity.

The food composition includes all forms such as functional food, nutritional supplement, health food, and food additives, and the types of food composition may be prepared in various forms according to a conventional method known in the art.

When the strain is used as a food additive, the strain may be added as it is or may be used with other food or food ingredients, and may be appropriately used according to a conventional method. The mix amount of the active ingredient may be appropriately determined depending on the purpose of use (prevention, health, or therapeutic treatment). In general, when prepare food or beverage, the active ingredient may be added in an amount of 0.0001 wt% to 1 wt%, specifically 0.001 wt% to 0.1 wt% in the raw material composition containing the strain. However, in the case of long-term intake for health and hygiene or health control purposes, the amount may be below the above range.

Yet another aspect of the present invention provides a feed composition for preventing or inhibiting a metabolic disease including a *Lactobacillus fermentum* strain (Accession No. KCTC 14105BP) or a culture thereof.

The *Lactobacillus fermentum* strain (Accession No. KCTC 14105BP) is the same as described above.

The feed composition for preventing or ameliorating a metabolic disease may be prepared by adding a *Lactobacillus fermentum* strain (Accession No. KCTC 14105BP) in an appropriate effective concentration range according to various methods for preparing a feed composition known in the art.

Yet still another aspect of the present invention provides a method of preventing and treating a metabolic disease including administering a *Lactobacillus fermentum* strain (Accession No. KCTC 14105BP) or a culture thereof to an individual.

The individual may have a metabolic disease. In addition, the individual may be a mammal, preferably a human. In this case, the *Lactobacillus fermentum* strain (Accession No. KCTC 14105BP) is the same as described above. In addition, the route of administration, dosage, and frequency of administration of the strain or a culture thereof may be administered to a subject in various ways and amounts depending on the condition of a patient, and the presence or absence of side effects, and optimal method of administration, dosage and frequency of administration may be appropriately selected within an appropriate range by a person skilled in the art. In addition, the types of metabolic diseases are as described above.

Yet another aspect of the present invention provides a use of a *Lactobacillus fermentum* strain (Accession No. KCTC 14105BP) or a culture thereof to treat a metabolic disease.

In this case, the *Lactobacillus fermentum* strain (Accession No. KCTC 14105BP) is the same as described above. In addition, a metabolic disease is as described above.

### Mode for Carrying out the Invention

Hereinafter, the present invention will be described in more detail by way of the following examples. However, the following examples are only for illustrating the present invention, and the scope of the present invention is not limited thereto.

### Example 1. Screening for a strain having anti-obesity effect

Lactic acid bacteria having anti-obesity efficacy among 16 lactic acid bacteria purchased from Mediogen Co., were selected using a mouse model. Specifically, C57BL/6 mice which were fed with 60% high fat diet (HFD), and then administered orally with the respective lactic acid bacteria, were used as an experimental group. Mice only fed with 60% high fat diet were used as a control group. In this case, each of lactic acid bacteria was orally administered at 5×10⁹ CFU per mouse daily. The anti-obesity effect was determined based on the body weight differences between the experiment group and the control group.

As a result, the *Lactobacillus fermentum GB102* strain most effectively inhibited body weight gain. In this case, there were some of *L. fermentum* lactic acid bacteria that did not exhibit anti-obesity effect (FIGS. 1 to 3). Based on this, it was confirmed that the *Lactobacillus fermentum GB102* strain exhibited anti-obesity effect among *L. fermentum* lactic acid bacteria.

### Example 2. Confirmation of fat accumulation inhibitory effect of L. fermentum GB102 strain in white adipose tissue

Using a mouse model, fat accumulation inhibitory effect of a *L. fermentum GB102* (Accession No. KCTC 14105BP) strain in white adipose tissue was confirmed. Specifically, C57BL/6 mice were fed with 60% high fat diet (HFD), and then the *L. fermentum GB102* strain was orally administered, which was used as an experimental group. In addition, mice fed only with 60% high fat diet were used as a negative control group, and mice fed with 60% high fat diet and oral administration of general lactic acid bacteria (*L. plantarum MG5120*) were used as a positive control group. Furthermore, mice fed with normal chow diet (NCD) were used as a normal group. In this case, general lactic acid bacteria purchased from Mediogen Co., or a *L. fermentum GB102* strain was orally administered daily at 5×10⁹ CFU per mouse.

After the administration of each test substance was completed, white adipose tissue was separated by autopsy. The separated white adipose tissue was fixed with 10% neutral buffered formalin. Then, paraffin sections were prepared, stained with Hematoxylin & Eosin (H&E), and then the size of the cells was observed.

The result showed that, while the size of the adipocytes in the negative and positive control groups fed with high fat diet was increased compared to the normal group, the size of the adipocytes in the experimental group to which a *L. fermentum GB102* strain was orally administered was significantly decreased (FIG. 4). Based on this, it was confirmed that the *L. fermentum GB102* strain effectively inhibits fat accumulation in white adipose tissue.

### Example 3. Confirmation of immune cell regulatory effect of L. fermentum GB102 strain in white adipose tissue

Using a mouse model, changes of immune cells caused by administration of a *L. fermentum GB102* strain in white adipose tissue were confirmed. Specifically, C57BL/6 mice which were fed with 60% (HFD), and then administered orally with a *L. fermentum GB102* strain, were used as an experimental group. In addition, mice fed only with 60% high fat diet were used as a negative control group, and mice fed with 60% high fat diet and oral administration of general lactic acid bacteria (*L*. *plantarum MG5120*) were used as a positive control group. Furthermore, mice fed with normal chow diet (NCD) were used as a normal group. In this case, general lactic acid bacteria purchased from Mediogen Co., or a *L. fermentum GB102* strain was orally administered daily at 5×10⁹ CFU per mouse.

After the administration of each test substance was completed, immune cells were separated from epididymal white adipocyte tissue of mice in each group by autopsy, stained the cells with specific antibodies, and then analyzed by flow cytometry. The adipose tissue was finely cut with curved scissors, cultured with 2 mg/mL collagen Type II (Worthington) for 45 minutes at 37°C, and then stromal vascular fraction (SVF) was isolated by a centrifuge. After washed with FACS buffer and then blocked at 4°C for 10 minutes to inhibit non-specific binding. Anti-MHCII (Biolegend, CA, USA), anti-F4/80 (Biolegend, CA, USA), anti-CD206 (Biolegend, CA, USA), anti-CDllc (Biolegend, CA, USA), and anti-CDllb (Biolegend, CA, USA) antibodies were added to M1/M2 macrophages, and allowed to react at 4°C for 30 minutes, followed by washing with FACS buffer. Treg cells were subjected to the same procedure as above using anti-TCRβ (Biolegend, CA, USA) and anti-CD4 (Biolegend, CA, USA) antibodies, and then treated with EBIOSCIENCE^{™} Foxp3/Transcription Factor Staining Buffer for fixation and increase of permeability to stain with an anti-Foxp3 antibody (eBioscience, CA, USA). Among MHCII+F4/80+ macrophages, CD11b+CD206+ was analyzed with M2 macrophages, and CD11b+CD11c+ with M1 macrophages. TCRβ+CD4+Foxp3+ were analyzed with Treg cells.

The result showed that the inflammatory M1 macrophages in the negative and positive control group mice fed with high fat diet were higher than that of the normal group, while the M1 macrophages in the experimental group mice to which the *L. fermentum GB102* strain was orally administered were significantly lower than that of the negative control group. It was also confirmed that anti-inflammatory M2 macrophages and Treg cells in the negative and positive control group mice fed with high fat diet were lower than that of the normal group, while M2 macrophages and Treg cells in the experimental group mice to which the *L. fermentum GB102* strain was administered orally were significantly increased compared to the negative control group. Based on this, immune cell regulatory effect of the *L. fermentum GB102* strain in white adipose tissue was confirmed (FIG. 5).

### Example 4. Confirmation of fat accumulation inhibitory effect of L. fermentum GB102 strain in brown adipose tissue

Using a mouse model, fat accumulation inhibitory effect of a *L. fermentum GB102* strain in brown adipose tissue was confirmed. Specifically, C57BL/6 mice which were fed with 60% (HFD), and then administered orally with a *L. fermentum GB102* strain, were used as an experimental group. In addition, mice fed only with 60% high fat diet were used as a negative control group, and mice fed with 60% high fat diet and oral administration of general lactic acid bacteria (*L. plantarum MG5120*) were used as a positive control group. Furthermore, mice fed with normal chow diet (NCD) were used as a normal group. In this case, general lactic acid bacteria purchased from Mediogen Co., or a *L. fermentum GB102* strain was orally administered daily at 5×10⁹ CFU per mouse.

After the administration of each test substance was completed, brown adipose tissue was separated from the mice in each group by autopsy. The separated brown adipose tissue was fixed with 10% neutral buffered formalin. Then, paraffin sections were prepared, stained with H&E, and then the size of the cells was observed.

The result showed that the size of the adipocytes in the negative and positive control groups mice fed with high fat diet was increased compared to the normal group, while the size of the adipocytes in the experimental group to which the *L. fermentum GB102* strain was administered orally was significantly decreased (FIG. 6). Based on this, it was confirmed that the *L. fermentum GB102* strain effectively inhibits fat accumulation in brown adipose tissue.

### Example 5. Confirmation of fat accumulation inhibitory effect of L. fermentum GB102 strain in liver tissue

Using a mouse model, fat accumulation inhibitory effect of a *L. fermentum GB102* strain in liver tissue was confirmed. Specifically, C57BL/6 mice which were fed with 60% (HFD), and then administered orally with the *L. fermentum GB102* strain, were used as an experimental group. In addition, mice fed only with 60% high fat diet were used as a negative control group, and mice fed with 60% high fat diet and oral administration of general lactic acid bacteria (*L. plantarum MG5120*) were used as a positive control group. Furthermore, mice fed with normal chow diet (NCD) were used as a normal group. In this case, general lactic acid bacteria purchased from Mediogen Co., or *a L. fermentum GB102* strain was orally administered daily at 5×10⁹ CFU per mouse.

After the administration of each test substance was completed, the liver was extracted by autopsy, and the weight was measured to compare and evaluate the weight between groups. After the extracted liver was fixed with 10% buffered formalin, paraffin sections were prepared, stained with H&E, and then the size of the cells was observed.

The result showed that the liver weight of the negative and positive control group mice fed with high fat diet were increased compared to the normal group, while the liver weight in the experimental group mice to which the *L. fermentum GB102* strain was administered orally were significantly decreased compared to the negative control group (FIG. 7). Furthermore, compared to the normal group, fat accumulation in liver tissue was increased in the negative and positive control group mice fed with high fat diet, while little fat was accumulated in liver tissue of the experimental group mice to which the *L. fermentum GB102* strain administered orally, and fat accumulation level was similar to that of the normal group (FIG. 8). Based on this, it was confirmed that the *L. fermentum GB102* strain effectively inhibits the liver weight and fat accumulation in liver tissue.

### Example 6. Confirmation of fasting blood glucose-lowering and glucose tolerance improvement effects of L. fermentum GB102 strain

Glucose tolerance test (GTT) was performed to confirm glucose tolerance improvement effect of a *L. fermentum GB102* strain. First, C57BL/6 mice which were fed with 60% (HFD), and then, administered orally with a *L. fermentum GB102* strain, were used as an experimental group. In addition, mice fed only with 60% high fat diet were used as a negative control group, and mice fed with 60% high fat diet and oral administration of general lactic acid bacteria (*L. plantarum MG5120*) were used as a positive control group. Furthermore, mice fed with normal chow diet (NCD) were used as a normal group. In this case, general lactic acid bacteria purchased from Mediogen Co., or a *L. fermentum GB102* strain was orally administered daily at 5×10⁹ CFU per mouse.

To perform glucose tolerance test, mice were fasted for 16 hours or more, and then a glucose solution was injected intraperitoneally at a dose of 1 g/kg. Then, blood was collected from the tail vein of the mice after 0, 30, 60, 90, and 120 minutes, and blood glucose was measured using a blood glucose meter. In this case, blood glucose at 0 minutes refers to fasting blood glucose.

The result showed that the fasting blood glucose level was significantly increased in the negative and positive control group mice fed with high fat diet, compared to the normal group. Meanwhile, the fasting blood glucose level of the experimental group mice to which the *L. fermentum GB102* strain was administered orally was significantly decreased compared to the negative control group (FIG. 9).

In addition, it was confirmed that the blood glucose level after administration of glucose in the negative and positive control group mice fed with high fat diet was higher than that of the normal group, while the blood glucose level of the experimental group mice to which *the L. fermentum GB102* strain was administered orally was significantly decreased compared to the negative control group (FIG. 10). Based on this, it was confirmed that the *L. fermentum GB102* strain exhibits fasting blood glucose-lowering and glucose tolerance improvement effects.

### Example 7. Confirmation of insulin resistance-ameliorating effect of L. fermentum GB102 strain

Insulin tolerance test (ITT) was performed to confirm insulin resistance-ameliorating effect of a *L. fermentum GB102* strain. First, C57BL/6 mice which were fed with a 60% (HFD) and then, administered orally with a *L. fermentum GB102* strain were used as an experimental group. In addition, mice fed only with 60% high fat diet were used as a negative control group, and mice fed with 60% high fat diet and oral administration of general lactic acid bacteria (*L. plantarum MG5120*) were used as a positive control group. Furthermore, mice fed with normal chow diet (NCD) were used as a normal group. In this case, general lactic acid bacteria purchased from Mediogen Co., or *L. fermentum GB102* strain was orally administered daily at 5×10⁹ CFU per mouse.

To perform insulin tolerance test, mice were fasted for 4.5 hours, and then an insulin solution was administered intraperitoneally at a dose of 1 U/kg. Blood was collected from the tail vein of the mice after 0, 30, 60, 90 and 120 minutes, and then blood glucose was measured with a blood glucose meter.

The result confirmed that the blood glucose level after administration of insulin in the negative and positive control group mice fed with high fat diet was higher than that of the normal group, while the blood glucose level of the experimental group mice to which the *L. fermentum GB102* strain was administered orally was significantly decreased compared to the negative control group (FIG. 11). Based on this, it was confirmed that the *L. fermentum GB102* strain effectively ameliorates insulin resistance by increasing insulin sensitivity.

### Example 8. Confirmation of metabolic hormones and adipokines regulatory effect of L. fermentum GB102 strain

Metabolic hormones and adipokines regulatory effect caused by administration of a *L. fermentum GB102* strain was confirm using a mouse model. Specifically, C57BL/6 mice which were fed with 60% (HFD) and then, administered orally with a *L. fermentum GB102* strain were used as an experimental group. In addition, mice fed only with 60% high fat diet were used as a negative control group, and mice fed with 60% high fat diet and oral administration of general lactic acid bacteria (*L. plantarum MG5120*) were used as a positive control group. Furthermore, mice fed with normal chow diet (NCD) were used as a normal group. In this case, general lactic acid bacteria purchased from Mediogen Co., or a *L. fermentum GB102* strain was orally administered daily at 5×10⁹ CFU per mouse. Serum was isolated from blood of the mice in each group, and GLP-1 and inflammatory adipokines in serum were analyzed by using a Bio-plex Pro Mouse Diabetes 8-plex assay kit (Bio-rad, Hercules, CA, USA) and Bioplex 200 (Bio-rad, Hercules, CA, USA). The sample analysis procedure was performed by referring to the protocol in the Kit.

The result showed that the concentration of GLP-1 in serum of the negative and positive control group mice fed with high fat diet was lower or similar compared to the normal group, while the concentration of GLP-1 of the experimental group mice to which the *L. fermentum GB102* strain was administered orally was significantly higher than the negative control group. In addition, the concentrations of PAI-1 and resistin in serum of the negative and positive control group mice fed with high fat diet were higher than that of the normal group, while the concentrations in the experimental group mice to which the *L. fermentum GB102* strain was administered orally were significantly decreased compared to the negative control group. Based on this, it was confirmed that the *L. fermentum GB102* strain increases the concentration of metabolic hormones in blood and decreases the concentration of inflammatory adipokines (FIG. 12).

### <Accession No.>

Depository institution Korea Research Institute of Bioscience and Biotechnology
Accession No. : KCTC14105BP
Date of Deposit: 20200114.

## Claims

1. A *Lactobacillus fermentum* strain (Accession No. KCTC 14105BP).

2. A pharmaceutical composition for preventing or treating a metabolic disease comprising, as an active ingredient, a *Lactobacillus fermentum* strain (Accession No. KCTC 14105BP) or a culture thereof.

3. The pharmaceutical composition for preventing or treating a metabolic disease of claim 2, wherein the metabolic disease is any one selected from the group consisting of obesity, hypertension, arteriosclerosis, hyperlipidemia, fatty liver, non-alcoholic fatty liver disease, hyperinsulinemia, diabetes, and insulin resistance syndrome.

4. The pharmaceutical composition for preventing or treating a metabolic disease of claim 2, wherein the strain inhibits fat accumulation in white adipose tissue.

5. The pharmaceutical composition for preventing or treating a metabolic disease of claim 2, wherein the strain reduces metabolic inflammation in white adipose tissue.

6. The pharmaceutical composition for preventing or treating a metabolic disease of claim 2, wherein the strain reduces fat accumulation in brown adipose tissue.

7. The pharmaceutical composition for preventing or treating a metabolic disease of claim 2, wherein the strain inhibits fat accumulation in liver tissue.

8. The pharmaceutical composition for preventing or treating a metabolic disease of claim 2, wherein the strain reduces a blood glucose level.

9. The pharmaceutical composition for preventing or treating a metabolic disease of claim 2, wherein the strain reduces insulin resistance.

10. The pharmaceutical composition for preventing or treating a metabolic disease of claim 2, wherein the strain regulates the concentration of metabolic hormones and adipokines in blood.

11. The pharmaceutical composition for preventing or treating a metabolic disease of claim 10, wherein the metabolic hormone is GLP-1.

12. The pharmaceutical composition for preventing or treating a metabolic disease of claim 10, wherein the adipokine is PAI-1 or resistin.

13. A food composition for preventing or inhibiting a metabolic disease comprising a *Lactobacillus fermentum* strain (Accession No. KCTC 14105BP) or a culture thereof.

14. A feed composition for preventing or inhibiting a metabolic disease comprising a *Lactobacillus fermentum* strain (Accession No. KCTC 14105BP) or a culture thereof.

15. A method of preventing and treating a metabolic disease comprising administering a *Lactobacillus fermentum* strain (Accession No. KCTC 14105BP) or a culture thereof to an individual.
